# FASCICULE DE BREVET EUROPEEN

(11) **EP 0 593 711 B1**
(45) Date de publication et mention de la délivrance du brevet: **12.06.1996**
(21) Numéro de dépôt: 93909002.3
(22) Date de dépôt: 07.04.1993
(51) Int. Cl.: A61K 7/13

(54) **PROCEDE DE TEINTURE DES FIBRES KERATINIQUES EN MILIEU ALCALIN METTANT EN OEUVRE DES PARAAMINOPHENOLS SUBSTITUES EN POSITION 2 EN ASSOCIATION AVEC LE 6- OU 7-HYDROXYINDOLE ET COMPOSITIONS MISES EN OEUVRE DANS LE PROCEDE**
VERFAHREN ZUR FÄRBUNG VON KERATINFASERN IN ALKALISCHEM MILIEU MIT HILFE VON 2-SUBSTITUIERTEN PARA-AMINOPHENOLEN IN KOMBINATION MIT 6- ODER 7- HYDROXYINDOL, UND ENTSPRECHENDE ZUSAMMENSETZUNGEN
METHOD FOR DYEING KERATIN FIBRES IN AN ALKALINE MEDIUM BY MEANS OF 2-SUBSTITUTED PARA-AMINOPHENOLS COMBINED WITH 6- OR 7-HYDROXYINDOLE, AND COMPOSITIONS THEREFOR

(30) Priorité: 09.04.1992 FR 9204346
(43) Date de publication de la demande: 27.04.1994
(73) Titulaire: L'OREAL, F-75008 Paris (FR)
(72) Inventeur: COTTERET, Jean, F-78480 Verneuil-sur-Seine (FR); AUDOUSSET, Marie, Pascale, F-92300 Levallois-Perret (FR)
(74) Mandataire: Casalonga, Axel
(86) Numéro de dépôt international: FR9300349
(87) Numéro de publication internationale: WO9320794

(56) Documents cités:
- EP-A- 0 359 618
- EP-A- 0 395 837
- EP-A- 0 446 132
- FR-A- 2 636 235
- FR-A- 2 636 236

## Description

La présente invention est relative à un nouveau procédé de teinture des fibres kératiniques et en particulier les fibres kératiniques humaines, mettant en oeuvre des paraaminophénols substitués en position 2 en association avec le 6-ou 7-hydroxyindole et un agent oxydant en milieu alcalin, et aux compositions mises en oeuvres au cours de ce procédé.

Il est connu de teindre les fibres kératiniques et en particulier les cheveux humains, avec des compositions tinctoriales contenant des précurseurs de colorants d'oxydation, en particuliers des ortho ou paraphénylènediamines des ortho ou paraaminophénols, généralement appelés "bases d'oxydation", et des coupleurs encore appelés modificateurs de coloration, plus particulièrement des métaphénylènediamines aromatiques, des métaaminophénols et des métadiphénols, qui permettent de modifier et d'enrichir de reflets les colorations" de fond "obtenues par les produits de condensation des bases d'oxydation.

On recherche, dans le domaine de la teinture capillaire d'oxydation, des précurseurs de colorants d'oxydation ainsi que des coupleurs qui permettent de conférer aux cheveux une coloration ayant une résistance satisfaisante à la lumière, aux lavages, aux intempéries, à la transpiration et aux différents traitements que peuvent subir les cheveux, et d'obtenir un large éventail de nuances de coloration.

Les paraaminophénols substitués en position 2, ainsi que leur utilisation dans des compositions tinctoriales pour fibres kératiniques en association avec des coupleurs classiques de type benzénique sont connus et décrits dans le brevet FR 2 637 282.

Un procédé de teinture pour la teinture des fibres kératiniques en milieu acide est décrit dans le brevet FR 2 659 228 de la demanderesse, comme mettant en oeuvre une composition contenant, à titre de coupleur, le 6-hydroxyindole, le 7-hydroxyindole ou leurs dérivés avec des précurseurs de colorant d'oxydation tels que les paraaminophénols. Toutefois un tel procédé ne procurait pas, après application sur les fibres, une coloration suffisamment résistante.

Dans le brevet FR 2 636 236, la demanderesse décrit des compositions tinctoriales pour la teinture des fibres kératiniques en milieu alcalin, contenant le 6-hydroxyindole ou le 7-hydroxyindole en tant que coupleur associé a une base d'oxydation dont le paraaminophénol et certains de ses dérivés. Les colorations obtenues avec de telles compositions ne présentaient pas une résistance satisfaisante notamment à la lumière.

La demanderesse vient de découvrir, ce qui fait l'objet de l'invention, que l'utilisation de certains paraaminophénols substitués en position 2 à titre de précurseurs de colorants d'oxydation en association avec le 6-hydroxyindole ou le 7-hydroxyindole et d'un agent oxydant permet d'obtenir en milieu alcalin, après application sur les fibres kératiniques et en particulier les cheveux humains, un large éventail de colorations aux nuances chaudes présentant une résistance à la lumière, aux lavages, aux intempéries, à la transpiration et aux différents traitements que peuvent subir les cheveux, particulièrement remarquable et supérieure à celles de l'art antérieur.

La présente invention a donc pour objet un procédé de teinture des fibres kératiniques, en particulier des fibres kératiniques humaines telles que les cheveux, comprenant l'application sur ces fibres d'au moins à titre de précurseur de colorant d'oxydation, un paraaminophénol substitué en position 2 de formule (I) ci-après, à titre de coupleur, le 6-hydroxyindole ou le 7-hydroxyindole et un agent oxydant, à pH alcalin.

L'invention a également pour objet un agent de teinture à deux composants, dont l'un des composants comprend le précurseur de colorant d'oxydation de formule (I) définie ci-après et le 6- ou 7-hydroxyindole, et l'autre l'agent oxydant.

Un autre objet de l'invention porte sur la composition prête à l'emploi, contenant les différents agents utilisés pour la teinture, en milieu alcalin, des fibres kératiniques.

L'invention a également pour objet des nécessaires de teinture ou "kits", à plusieurs composants, permettant de mettre en oeuvre le procédé indiqué ci-dessus.

D'autres objets de l'invention apparaîtront à la lecture de la description et des exemples qui suivent.

Le procédé de teinture des fibres kératiniques et en particulier des fibres kératiniques humaines telles que les cheveux, conforme à l'invention est essentiellement caractérisé par le fait que l'on applique sur ces fibres au moins un paraaminophénol substitué en position 2 de formule (I) 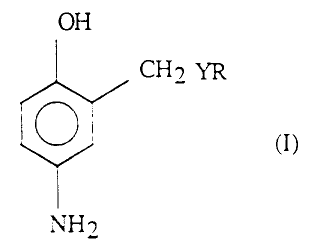
dans laquelle :
Y représente un atome d'oxygène ou un atome de soufre, R représente un radical alkyle en C₁-C₄, monohydroxyalkyle en C₁-C₄ ou polyhydroxyalkyle en C₂-C₄, ainsi que leurs sels d'addition avec un acide;
le 6-hydroxyindole et/ou le 7-hydroxyindole;
la couleur étant révélée à pH supérieur à 7, à l'aide d'un agent oxydant.

Selon le procédé conforme à l'invention, on applique sur les fibres kératiniques et en particulier les fibres kératiniques humaines, au moins une composition (A) contenant dans un milieu approprié pour la teinture, au moins un paraaminophénol substitué en position 2 de formule (I) définie ci-dessus à titre de précurseur de colorant d'oxydation, en association avec le 6-hydroxyindole et/ou le 7-hydroxyindole à titre de coupleur, la couleur étant révélée à pH alcalin, à l'aide d'un agent oxydant présent dans la composition (A) ou appliqué simultanément ou séquentiellement de façon séparée, au moyen d'une composition (B) le contenant dans un milieu approprié pour la teinture.

Parmi les significations préférées du radical R dans les composés paraaminophénols de formule générale (I), le radical alkyle en C₁-C₄ désigne les radicaux méthyle, éthyle, propyle, isopropyle, butyle, isobutyle, le radical mono ou polyhydroxyalkyle désigne

-CH₂-CH₂OH, -CH₂-CHOH-CH₂-OH, -CH₂-CHOH-CH₃

Les sels d'acide correspondant aux composés paraaminophénols de formule générale (I) sont choisis de préférence parmi les chlorhydrates, les sulfates, les bromhydrates ou les tartrates.

Parmi les paraaminophénols de formule générale (I), on peut citer les composés suivants :
- le 2-méthoxyméthyl 4-aminophénol,
- le 2-éthoxyméthyl 4-aminophénol,
- le 2-propoxyméthyl 4-aminophénol,
- le 2 isopropoxyméthyl 4-aminophénol,
- le 2-(β-hydroxyéthoxyméthyl) 4-aminophénol,
- le 2-méthylthiométhyl 4-aminophénol,
- le 2-(β-hydroxyéthylthiométhyl) 4-aminophénol,
- le 2-(β,γ-dihydroxypropylthiométhyl) 4-aminophénol,
et leurs sels,
Parmi les composés préférés de formule (I), on peut citer :
- le 2-méthoxyméthyl 4-aminophénol
- le 2-éthoxyméthyl 4-aminophénol
- le 2-(β-hydroxyéthoxyméthyl) 4-aminophénol
- le 2-méthylthiométhyl 4-aminophénol
et leurs sels,
Selon le procédé de l'invention, on utilise de préférence en association avec le paraaminophénol de formule générale (I) définie précédemment, le 6-hydroxyindole à titre de coupleur.

L'agent oxydant est choisi de préférence parmi le peroxyde d'hydrogène, le peroxyde durée, les bromates de métaux alcalins, les persels tels que les perborates et les persulfates. Le peroxyde d'hydrogène est particulièrement préféré.

La composition (A) qui renferme, à titre de précurseur de colorant d'oxydation, au moins un paraaminophénol de formule (I) et le 6-hydroxyindole et/ou le 7-hydroxyindole à titre de coupleur, peut avoir un pH compris entre 3 et 10,5 et peut être ajusté à la valeur choisie au moyen d'agents alcalinisants habituellement utilisés en teinture des fibres kératiniques, tels que l'ammoniaque, les carbonates alcalins, les alcanolamines tels que les mono-, di-et triéthanolamines ainsi que leurs dérivés, les hydroxydes de sodium ou de potassium, ou des agents acidifiants classiques, tels que les acides minéraux ou organiques, tels que les acides chlorhydrique, tartrique, citrique et phosphorique.

Le pH de la composition (B) renfermant l'agent oxydant tel que défini ci-dessus, est tel qu'après mélange avec la composition A, le pH du mélange soit supérieur à 7 et de préférence compris entre 8 et 11.

Les composés de formule (I) sont présents dans la composition appliquée sur les fibres kératiniques, dans des proportions comprises de préférence entre 0,05 et 3,5 % en poids par rapport au poids total de la composition; le 6-hydroxyindole et/ou le 7-hydroxyindole y sont présents dans des proportions comprises entre 0,01 et 4 % en poids par rapport au poids total de la composition.

Les compositions tinctoriales définies ci-dessus et mises en oeuvre dans le procédé de teinture de l'invention, peuvent également contenir, en plus du paraaminophénol de formule (I) définie ci-dessus, d'autres précurseurs de colorants d'oxydation para et/ou ortho connus en eux-mêmes.

Ces précurseurs de colorants d'oxydation de type ortho ou para peuvent être choisis parmi les paraphénylènediamines, les paraaminophénols différents de ceux de formule (I), les précurseurs hétérocycliques para dérivés de la pyridine ou de la pyrimidine, tels que la 2,5-diaminopyridine, la 2-hydroxy 5-aminopyridine, la 2,4,5,6-tétraaminopyrimidine, le 4,5-diamino 1-méthylpyrazole, la 2-diméthylamino 4,5,6-triaminopyrimidine, les orthoaminophénols et les bases dites "doubles".

A titre de paraphénylènediamines, on peut plus particulièrement citer les composés répondant à la formule (II) : 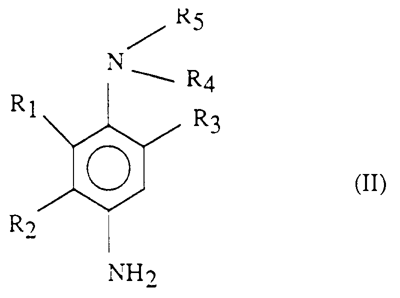
dans laquelle :
R₁, R₂, R₃, identiques ou différents, représentent un atome d'hydrogène ou d'halogène, un radical alkyle en C₁-C₄, un radical alcoxy en C₁-C₄, un radical carboxy, sulfo ou hydroxyalkyle en C₁-C₄;
R₄ et R₅, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino. ou bien R₄ et R₅ forment conjointement avec l'atome d'azote auquel ils sont liés, un hétérocycle pipéridino ou morpholino, sous réserve que R₁ ou R₃ représente un atome d'hydrogène lorsque R₄ et R₅ ne représentent pas un atome d'hydrogène, ainsi que les sels de ses composés. Ces groupes alkyle ou alcoxy ont de 1 à 4 atomes de carbone et désignent notamment méthyle, éthyle, propyle, méthoxy et éthoxy.

Parmi les composés de formule (II) on peut citer la paraphénylènediamine, la p-toluylènediamine, la méthoxyparaphénylènediamine, la chloroparaphénylènediamine, la 2,3-diméthylparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl) aniline, la 4-amino N,N-(éthyl, carbamylméthyl) aniline, la 3-méthyl 4-amino N,N-(éthyl, carbamylméthyl) aniline, la 4-amino N,N-(éthyl, β-pipéridinoéthyl) aniline, la 3-méthyl 4-amino N,N-(éthyl, β-pipéridinoéthyl)aniline, la 4-amino N,N-(éthyl, β-morpholinoéthyl)aniline la 3-méthyl 4-amino N,N-(éthyl,β-morpholinoéthyl) aniline, la 4-amino N,N-(éthyl, β-acétylaminoéthyl) aniline, la 4-amino N-(β-méthoxyéthyl) aniline, la 3-méthyl 4-amino N,N-(éthyl, β-acétylaminoéthyl) aniline, la 4-amino N,N-(éthyl, β-mésylaminoéthyl) aniline, la 3-méthyl 4-amino N,N-(éthyl, β-mésylamino-éthyl) aniline, la 4-amino N,N-(éthyl, β-sulfoéthyl)aniline, la 3-méthyl 4-amino N,N-(éthyl, β-sulfoéthyl) aniline, la N-[(4'-amino)phényl]-morpholine, la N-[(4'-amino)phényl]pipéridine, la 2-hydroxyéthylparaphénylènediamine, la fluoroparaphénylènediamine, la carboxyparaphénylènediamine la sulfoparaphénylènediamine, la 2-isopropylparaphénylènediamine, la 2-n-propylparaphénylènediamine, la 2-hydroxyméthylparaphénylènediamine, la N,N-diméthyl 3-méthyl paraphénylènediamine, la N, N-(éthyl,β hydroxyéthyl)paraphénylène diamine, la N-(dihydroxypropyl)para-phénylènediamine, la N-[(4'-amino)phényl]paraphénylènediamine, la N-(phényl)para phénylènediamine.

Ces paraphénylènediamines peuvent être introduites dans la composition tinctoriale, soit sous forme de base libre, soit sous forme de sels, tels que chlorhydrate, bromhydrate ou sulfate.

Parmi les paraaminophénols différents de ceux de formule (I), on peut citer le p-aminophénol, le 2-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol le 2-(β-hydroxyéthyl)4-aminophénol, le 2-méthoxy 4-aminophénol, le 3-méthoxy 4-aminophénol, le 3-(β-hydroxyéthoxy)4-aminophénol, le 2-aminométhyl 4-aminophénol le 2-β-hydroxyéthylaminométhyl 4-aminophénol.

Les bases dites "doubles"sont des bis-phénylalkylènediamines, répondant à la formule : 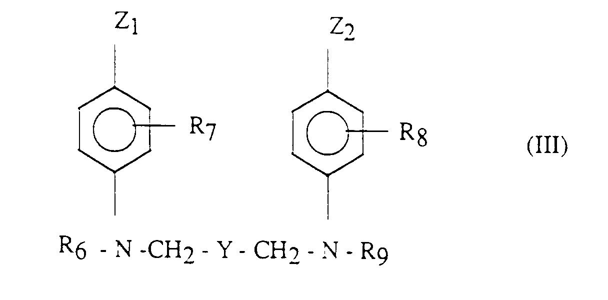
dans laquelle :
Z₁ et Z₂, identiques ou différents, représentent des groupements hydroxyle ou NHR₁₀, où R₁₀ désigne un atome d'hydrogène ou un radical alkyle inférieur ;
R₇ et R₈, identiques ou différents, représentent soit des atomes d'hydrogène, soit des atomes d'halogène, soit encore des radicaux alkyle ;
R₆ et R₉, identiques ou différents, représentent un atome d'hydrogène, un radical alkyle, hydroxyalkyle ou aminoalkyle, dont le reste amino peut être substitué;
Y' représente un radical pris dans le groupe constitué par les radicaux suivants :

-(CH₂)ₙ-,-(CH₂)ₘ-O-(CH₂)ₘ-, -(CH₂)_{q}-CHOH-(CH₂)_{q}-,

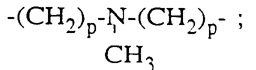

dans lesquels n est un nombre entier compris entre 0 et 8 et m, q et p sont des nombres entiers compris entre 0 et 4, cette base pouvant se présenter également sous forme de ses sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy et éthoxy.

Parmi les composés de formule (III) on peut citer le N,N'-bis-(β-hydroxyéthyl)N,N'-bis-(4'-aminophényl) 1,3-diamino 2-propanol, la N,N'bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthylaminophényl)tétraméthylènediamine, la N,N'-bis-(éthyl) N,N'-bis-(4'-amino 3'-méthylphényl)éthylènediamine.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxyhenzène, et les orthophénylènediamines.

Les compositions définies ci-dessus, appliquées dans la teinture des fibres kératiniques peuvent également contenir en plus du 6-hydroxyindole et/ou du 7-hydroxyindole utilisés comme coupleurs, d'autres coupleurs connus en eux-mêmes, tels que des métadiphénols, des métaaminophénols, des métaphénylènediamines, des métaacylaminophénols, des métauréidophénols, des métacarbalcoxyaminophénols, l'α-naphtol, des coupleurs possédant un groupement méthylène actif, tel que les composés β-cétoniques, les pyrazolones.

Parmi ces coupleurs on peut plus particulièrement citer, le 2, 4-dihydroxyphénoxyéthanol, le 2,4-dihydroxyanisole, le métaaminophénol, le monométhyléther de résorcine, la résorcine la 2-méthyl-résorcine, le 2-méthyl 5-aminophénol, le 2-méthyl 5-N-(β-hydroxyéthyl) aminophénol, le 2-méthyl 5-N-(β-mésylaminoéthyl)aminophénol, le 2,6-diméthyl 3-aminophénol, la 6-hydroxybenzomorpholine, le 2,4-diaminoanisole, le 2,4-diaminophénoxyéthanol, la 6-aminobenzomorpholine, le [2-(β-hydroxyéthyl) amino 4-amino]-phénoxyéthanol, le 2-amino 4-(β-hydroxyéthyl) amino anisole, le (2,4-diamino)phényl-β-γ-dihydroxypropyléther, la 2,4-diaminophénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chloro résorcinol, la 6-méthoxy 3-hydroxyéthylaminoaniline, le 1-éthoxy 2-bis(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3-aminophénol, le 6-chloro 3-trifluoroéthylaminophénol, et leurs sels.

On peut rajouter à ces compositions, comme cela est bien connu dans l'état de la technique, notamment en vue de nuancer ou d'enrichir en reflets les colorations apportées par le paraaminophénol associé au 6-hydroxyindole et/ou au 7-hydroxyindole, des colorants directs tels que des colorants azoïques, anthraquinoniques ou des dérivés nitrés de la série benzénique.

L'ensemble des précurseurs de colorants par oxydation de type para et/ou ortho, ainsi que les coupleurs utilisés dans les compositions tinctoriales mises en oeuvre dans le procédé selon l'invention, représente de préférence de 0,3 à 7 % en poids par rapport au poids de la dite composition.

Les compositions tinctoriales mises en oeuvre dans le procédé selon l'invention contiennent également dans leur forme de réalisation préférée, des agents tensio-actifs anioniques, cationiques, nonioniques, amphotères ou leurs mélanges. Parmi ces agents tensioactifs, on peut citer les alkylbenzènesulfonates, les alkylnaphtalènesulfonates, les sulfates, les éthersulfates et les sulfonates d'alcools gras, les sels d'ammonium quatemaires, tels que le bromure de triméthylcétylammonium, le bromure de cétylpyridinium, les éthanolamides d'acides gras éventuellement oxyéthylénés, les acides, alcools ou amines polyoxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, l'éthylèneglycol, le propylèneglycol, le monoéthyl éther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions mises en oeuvre dans le procédé selon l'invention peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique.

Ces agents antioxydants sont présents dans la composition dans des proportions comprises entre 0,05 et 1,5 % en poids par rapport au poids total de la composition.

Ces compositions peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants...

La composition appliquée sur les cheveux peut se présenter sous des formes diverses, telles que sous forme de liquide, de crème, de gel ou sous toute autre forme appropriée pour réaliser une teinture des fibres kératiniques et notamment des cheveux humains. Ces compositions peuvent être conditionnées sous pression en flacons aérosols en présence d'un agent propulseur et former des mousses.

L'invention a également pour objet, la composition prête à l'emploi utilisée dans le procédé défini ci-dessus.

Selon une forme de réalisation particulièrement préférée, le procédé comporte une étape préliminaire consistant à stocker sous forme séparée, d'une part, la composition contenant dans un milieu approprié pour la teinture au moins à titre de précurseur de colorant d'oxydation, un paraaminophénol répondant à la formule (I) définie ci-dessus et à titre de coupleur le 6-hydroxyindole et/ou le 7-hydroxyindole sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus, la composition résultante ayant une valeur de pH supérieure à 7 et de préférence comprise entre 8 et 11.

Conformément à l'invention, le procédé de teinture consiste à appliquer sur les cheveux le mélange obtenu, à le laisser poser pendant 10 à 40 minutes de préférence 15 à 30 minutes puis à rincer les cheveux , les laver au shampooing, les rincer à nouveau et les sécher.

Il est également possible, conformément à l'invention, d'appliquer séparément une composition contenant le paraaminophénol de formule (I), le 6-hydroxyindole et/ou le 7-hydroxyindole et l'agent oxydant qui peut être introduit, juste avant l'application, dans la composition appliquée dans le deuxième temps ou bien être additionné sur les fibres kératiniques dans un troisième temps, de façon à ce que le mélange se formant in-situ au niveau des fibres ait un pH supérieur à 7, les conditions de pose, de lavage et de séchage étant les mêmes que précédemment.

L'invention a également pour objet un agent de teinture des fibres kératiniques, en particulier des cheveux humains, essentiellement caractérisé par le fait qu'il comporte au moins deux composants, l'un des composants étant constitué par la composition (A) définie ci-dessus et l'autre étant constitué par la composition (B) également définie ci-dessus, le pH des compositions (A) et (B) étant tel qu'après mélange dans des proportions de 90 à 10 % pour la composition (A) et de 10 à 90 % pour la composition (B), la composition résultante ait un pH supérieur à 7, et de préférence compris entre 8 et 11.

La composition appliquée sur les fibres kératiniques résulte en particulier d'un mélange de 10 à 90 % du composant (A) avec 90 à 10 % du composant (B) contenant un agent oxydant et a un pH supérieur à 7 et de préférence compris entre 8 et 11.

Cet agent de teinture à deux composants peut être conditionné dans un dispositif à plusieurs compartiments ou kit de teinture qui constitue un autre objet de l'invention, ou tout autre système de conditionnement à plusieurs compartiments dont l'un des compartiments renferme le composant (A) et le second compartiment renferme le composant (B); ces dispositifs pouvant être équipés d'un moyen permettant de délivrer sur les cheveux le mélange souhaité, tel que les dispositifs décrits plus particulièrement dans le brevet US-A-4 823 985 de la demanderesse.

Les exemples qui suivent sont destinés à illustrer l'invention sans pour autant présenter un caractère limitatif.

### EXEMPLES 1 à 5

On procède à la teinture des cheveux en appliquant sur des cheveux permanentés ou non permanentés, gris à 90 % de blancs, un mélange extemporané de la composition colorante (A) et de la composition oxydante (B).

On laissse agir ce mélange pendant 30 minutes, puis on rince les cheveux, on effectue un shampooing. Après séchage, les cheveux sont teints dans la nuance précisée au bas du tableau ci-après.

| en g | 1 | 2 | 3 | 4 |
|---|---|---|---|---|
| A) Composition colorante | | | | |
| 6-hydroxindole | 0,532 | 0,798 | 0,266 | |
| 7-hydroxyindole | | | | 0,532 |
| 2-méthoxyméthyl 4-aminophénol | 0,436 | 0,918 | 0,918 | 0,612 |
| 3-amino 6-méthyl phénol | | | 0,246 | |
| 6-(β-hydroxyéthoxy) 1,3-diaminobenzène | | | 0,482 | |
| Support 1 | | | X | X |
| Support 2 | X | X | | |
| Eau qsp | 100 | 100 | 100 | 100 |

| B) Composition oxydante | | | | |
|---|---|---|---|---|
| Solution d'eau oxygénée à 20 volumes | 100 | 100 | 100 | 100 |
| Acide phosphorique qs pH | 3 | 3 | 3 | 3 |
| mélange p/p 1/3 A + 2/3B | | | X | |
| mélange p/p A + B | X | X | | X |
| pH du mélange | 9,8 | 9,8 | 9,6 | 9,8 |
| Nuances obtenues sur cheveux 90 % blancs permanentés | bond doré cuivré | blond foncé cuivré | blond foncé marron rouge cuivré | blond irisé puissant |

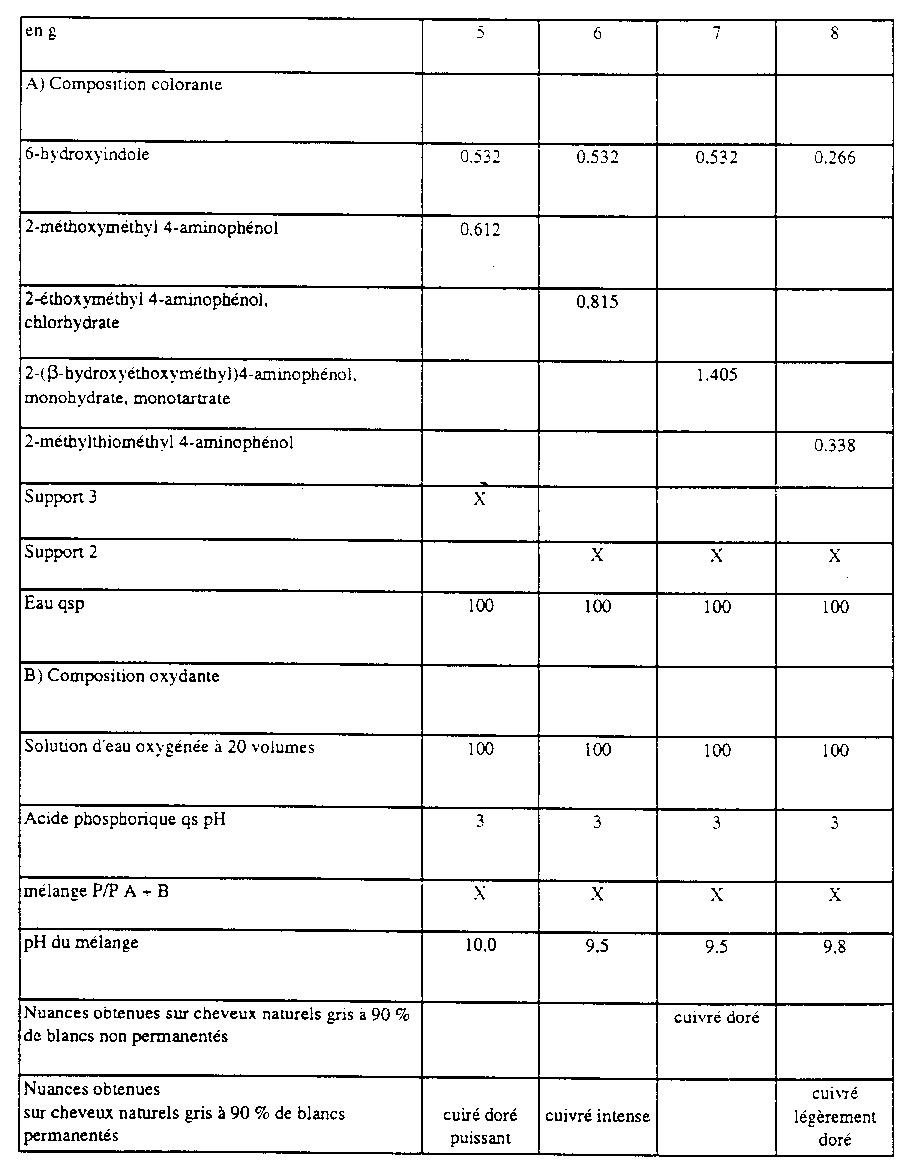

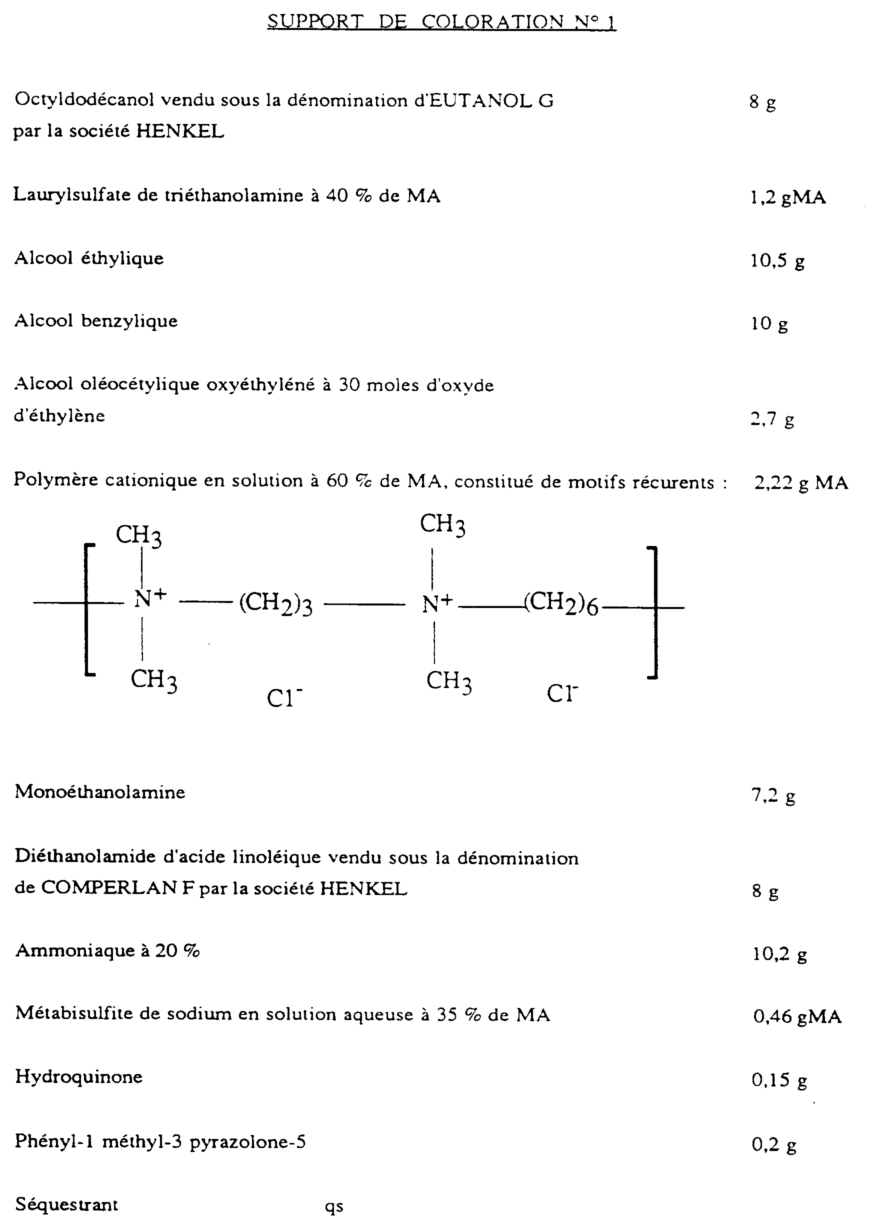

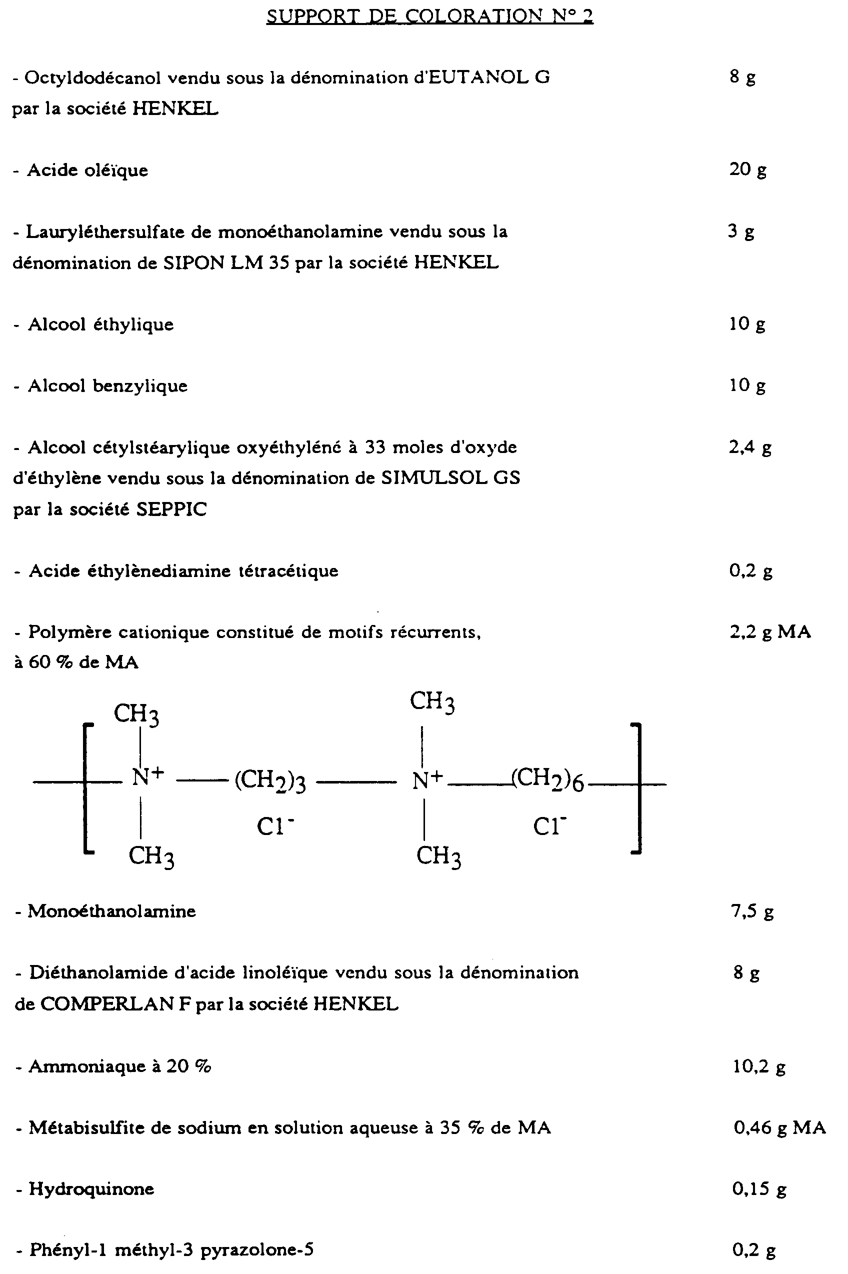

| SUPPORT DE COLORATION N°3 | |
|---|---|
| - Alcool oléique polyglycérolé à 2 moles de glycérol | 4 g |
| - Alcool oléique polyglycérolé à 4 moles de glycérol à 78 % de MA | 5,7 g MA |
| - Acide oléique | 3,0 g |
| - Amine oléique oxyéthylénée, à 2 moles d'oxyde d'éthylène, vendue sous la dénomination ETHOMEEN 0 12 par la société AKZO | 7 g |
| - Laurylamino succinamate de diéthylaminopropyle, sel de sodium, à 55 % de MA | 3 g MA |
| - Alcool oléique | 5 g |
| - Diéthanolamide d'acide de coprah | 12 g |
| - Propylène glycol | 3,5 g |
| - Dipropylèneglycol | 0,5 g |
| - Alcool éthylique | 7,0 g |
| - Monométhyl éther de propylène glycol | 9 g |
| - Métabisulfite de sodium en solution aqueuse à 35 % de MA | 0,46 g MA |
| - Acétate d'ammonium | 0,8 g |
| - monoéthanolamine qs pH = 9,8 | |
| - Antioxydant, séquestrant qs | |

## Revendications

1. Procédé de teinture des fibres kératiniques, en particulier des fibres kératiniques humaines, telles que les cheveux caractérisé par le fait que l'on applique sur ces fibres au moins un paraaminophénol substitué en position 2 de formule : 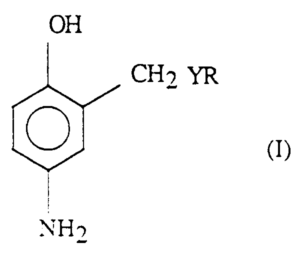 dans laquelle,
Y représente un atome d'oxygène ou un atome de soufre,
R représente un radical alkyle en C₁-C₄, monohydroxyalkyle en C₁-C₄ ou polyhydroxyalkyle en C₂-C₄, ainsi que leurs sels d'addition avec un acide; et, le 6-hydroxyindole et/ou le 7-hydroxyindole;
la couleur étant révélée à pH alcalin et à l'aide d'un agent oxydant.

2. Procédé selon la revendication 1, caractérisé en ce que l'on applique sur les fibres kératiniques, en particulier les fibres kératiniques humaines, au moins une composition (A) contenant dans un milieu approprié pour la teinture au moins, à titre de précurseur de colorant d'oxydation, un paraaminophénol substitué en position 2 de formule (I) et le 6-hydroxyindole et/ou 7-hydroxyindole à titre de coupleur;
la couleur étant révélée à pH alcalin et à l'aide d'un agent oxydant présent dans la composition (A) ou appliqué simultanément ou séquentiellement de façon séparée, au moyen d'une composition (B) le contenant dans un milieu approprié pour la teinture;

3. Procédé selon la revendication 1 ou 2, caractérisé par le fait que l'agent oxydant est choisi parmi le peroxyde d'hydrogène, le peroxyde d'urée, les bromates de métaux alcalins et les persels.

4. Procédé selon l'une quelconque des revendications 1 à 3, caractérisé en ce que dans les composés de formule (I) R désigne un radical méthyle, éthyle, propyle, iso-propyle, butyle, isobutyle, un radical mono-ou polyhydroxyalkyle choisi parmi :
CH₂-CH₂-OH, -CH₂-CHOH-CH₂-OH, -CH₂-CHOH-CH₃,

5. Procédé selon l'une quelconque des revendications 1 à 4, caractérisé en ce que les composés de formule (I) sont choisis parmi les composés suivants :
- le 2-méthoxyméthyl 4-aminophénol,
- le 2-éthoxyméthyl 4-aminophénol,
- le 2-propoxyméthyl 4-aminophénol,
- le 2 isopropoxyméthyl 4-aminophénol
- le 2-(β-hydroxyéthoxyméthyl) 4-aminophénol,
- le 2-méthylthiométhyl 4-aminophénol,
- le 2-(β-hydroxyéthylthiométhyl) 4-aminophénol,
- le 2-(β,γ-dihydroxypropylthiométhyl) 4-aminophénol,
et leurs sels.

6. Procédé selon l'une quelconque des revendications 1 à 5 caractérisé en ce que l'on applique sur les fibres kératiniques le 6-hydroxyindole à titre de coupleur.

7. Procédé selon l'une quelconque des revendications 1 à 6, caractérisé en ce que le pH de la ou des composition (s) appliquée (s) sur les fibres kératiniques est tel que la valeur du pH finale au niveau des fibres est comprise entre 8 et 11.

8. Procédé selon l'une quelconque des revendications 2 à 7, caractérisé en ce que la composition (A) contient 0,05 à 3,5 % en poids du poids total de la composition d'au moins un composé de formule (I).

9. Procédé selon l'une quelconque des revendications 2 à 8, caractérisé en ce que la composition (A) contient de 0,01 à 4 % en poids par rapport au poids total de la composition de 6-hydroxyindole et/ou 7-hydroxyindole.

10. Procédé selon l'une quelconque des revendications 2 à 9, caractérisé par le fait que la composition (A) contient outre le paraaminophénol de formule (I) définie ci-dessus, d'autres précurseurs de colorant d'oxydation de type ortho ou para choisis parmi les paraphénylènediamines, les paraaminophénols différents de ceux de formule (I), les précurseurs hétérocycliques para dérivés de la pyridine ou de la pyrimidine, les orthoaminophénols les orthophénylènediamines et les bis-phénylalkylènediamines.

11. Procédé selon l'une quelconque des revendications 2 à 10, caractérisé en ce que la composition (A) contient outre le 6-hydroxyindole et/ou le 7-hydroxyindole, d'autres coupleurs tels que des métaaminophénols, des métaphénylènediamines, des métaacylaminophénols, des métauréidophénols, des métacarbalcoxyaminophénols, l'α-naphtol, des coupleurs possédant un groupe méthylène actif.

12. Procédé selon l'une quelconque des revendications 2 à 11, caractérisé en ce que la composition (A) contient de 0,3 à 7 % en poids par rapport au poids total de la composition, de précurseur de colorant d'oxydation du type ortho et/ou para et de coupleur.

13. Procédé selon l'une quelconque des revendications 2 à 12, caractérisé en ce que la composition (A) contient des agents tensioactifs cationiques, anioniques, non-ioniques, amphotères ou leurs mélanges, en des concentrations comprises entre 0,5 % et 55 % en poids par rapport au poids total de la composition; des solvants organiques en des concentrations comprises entre 1 et 40 % en poids par rapport au poids total de la composition; des agents épaississants en des concentrations comprises entre 0,1 et 5 % en poids par rapport au poids total de la composition; des agents antioxydants en des proportions comprises entre 0,05 et 1,5 % en poids par rapport au poids total de la composition, des colorants directs, des parfums, des agents séquestrants, des agents filmogènes, des agents de traitement, des agents dispersants, des agents de conditionnement, des agents conservateurs des agents opacifiants et tout autre agent habituellement utilisés dans des compositions tinctoriales.

14. Procédé selon l'une quelconque des revendications 2 à 13, caractérisé par le fait que les compositions (A) et (B) se présentent sous forme de liquide, de crème, de gel ou toute autre forme appropriée, ou peuvent être conditionnées sous pression en flacon aérosol en présence d'un agent propulseur et former des mousses.

15. Procédé selon la revendication 1, caractérisé en ce que l'on applique sur les fibres kératiniques et plus particulièrement les cheveux humains, dans un premier temps, une composition contenant au moins un paraaminophénol de formule (I), dans un deuxième temps une composition contenant le 6-hydroxyindole et/ou le 7-hydroxyindole, et l'on développe la coloration à l'aide d'un agent oxydant présent dans cette dernière composition ou bien l'on applique cet agent oxydant dans un troisième temps, le pH du mélange se formant au niveau de la fibre étant supérieur à 7.

16. Procédé de teinture d'oxydation selon l'une quelconque des revendications 1 à 15, caractérisé par le fait qu'après l'application sur les fibres kératiniques, et plus particulièrement les cheveux humains, on laisse poser le mélange pendant 10 à 40 minutes, de préférence 15 à 30 minutes, on rince les cheveux, on les lave au shampoing, on les rince à nouveau et on les sèche.

17. Agent de teinture des fibres kératiniques, en particulier des cheveux humains, caractérisé par le fait qu'il comporte au moins deux composants, l'un des composants étant constitué par la composition (A) définie dans l'une quelconque des revendications 2 à 14 et l'autre étant constitué par la composition (B) également définie dans l'une quelconque des revendications 2 à 14, le pH des compositions (A) et (B) étant tel qu'après mélange dans des proportions de 90 à 10 % pour la composition (A) et de 10 à 90 % pour la composition (B), la composition résultante ait un pH supérieur à 7.

18. Composition destinée à être utilisée pour la coloration des fibres kératiniques à pH basique, caractérisée par le fait qu'elle résulte du mélange de 10 à 90 % de la composition (A) définie dans l'une quelconque des revendications 2 à 14 avec 90 à 10 % de la composition (B) définie dans l'une quelconque des revendications 2 à 14 et qu'elle a un pH supérieur à 7.

19. Dispositif à plusieurs compartiments ou kit de teinture, caractérisé par le fait qu'il comprend dans un premier compartiment la composition (A) définie dans l'une quelconque des revendications 2 à 14, et dans un second compartiment la composition (B) définie dans l'une quelconque des revendications 2 à 14.

## Claims

1. Process for dyeing keratinous fibres, in particular human keratinous fibres, such as hair, characterised in that there is applied to these fibres at least one para-aminophenol substituted in position 2 of formula: 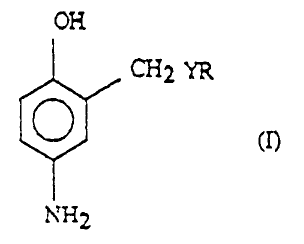 in which:
Y represents an oxygen atom or sulphur atom,
R represents a C₁-C₄ alkyl, C₁-C₄ monohydroxyalkyl or C₂-C₄ polyhydroxyalkyl radical, and their addition salts with an acid; and, 6-hydroxyindole and/or 7-hydroxyindole;
the colour being displayed at alkaline pH and using an oxidising agent.

2. Process according to Claim 1, characterised in that there is applied to keratinous fibres, in particular human keratinous fibres, at least one composition (A) containing, in a medium suitable for dyeing, at least, as oxidation dye precursor, one para-aminophenol substituted in position 2 of formula (I) and 6-hydroxyindole and/or 7-hydroxyindole as coupler;
the colour being displayed at alkaline pH and using an oxidising agent present in the composition (A) or applied separately simultaneously or sequentially by means of a composition (B) containing it in a medium suitable for dyeing.

3. Process according to Claim 1 or 2, characterised in that the oxidising agent is chosen from hydrogen peroxide, urea hydrogen peroxide, alkali metal bromates and persalts.

4. Process according to any one of Claims 1 to 3, characterised in that, in the compounds of formula (I), R denotes a methyl, ethyl, propyl, iso-propyl, butyl or isobutyl radical or a mono- or polyhydroxyalkyl radical chosen from:
CH₂-CH₂-OH, -CH₂-CHOH-CH₂-OH or -CH₂-CHOH-CH₃.

5. Process according to any one of Claims 1 to 4, characterised in that the compounds of formula (I) are chosen from the following compounds:
- 2-methoxymethyl-4-aminophenol,
- 2-ethoxymethyl-4-aminophenol,
- 2-propoxymethyl-4-aminophenol,
- 2-isopropoxymethyl-4-aminophenol,
- 2-(β-hydroxyethoxymethyl)-4-aminophenol,
- 2-methylthiomethyl-4-aminophenol,
- 2-(β-hydroxyethylthiomethyl)-4-aminophenol,
- 2-(β,γ-dihydroxypropylthiomethyl)-4-aminophenol,
and their salts.

6. Process according to any one of Claims 1 to 5, characterised in that 6-hydroxyindole is applied to keratinous fibres as coupler.

7. Process according to any one of Claims 1 to 6, characterised in that the pH of the composition(s) applied to keratinous fibres is such that the final pH value of the fibres is between 8 and 11.

8. Process according to any one of Claims 2 to 7, characterised in that the composition (A) contains 0.05 to 3.5 % by weight of the total weight of the composition of at least one compound of formula (I).

9. Process according to any one of Claims 2 to 8, characterised in that the composition (A) contains from 0.01 to 4 % by weight, with respect to the total weight of the composition, of 6-hydroxyindole and/or 7-hydroxyindole.

10. Process according to any one of Claims 2 to 9, characterised in that the composition (A) contains, besides the para-aminophenol of formula (I) defined above, other oxidation dye precursors of ortho or para type chosen from para-phenylenediamines, para-aminophenols other than those of formula (I), para heterocyclic precursors derived from pyridine or from pyrimidine, ortho-aminophenols, ortho-phenylenediamines and bisphenylalkylenediamines.

11. Process according to any one of Claims 2 to 10, characterised in that the composition (A) contains, besides 6-hydroxyindole and/or 7-hydroxyindole, other couplers such as meta-aminophenols, meta-phenylenediamines, meta-acylaminophenols, meta-ureidophenols, meta-carbalkoxyaminophenols, α-naphthol or couplers having an active methylene group.

12. Process according to any one of Claims 2 to 11, characterised in that the composition (A) contains from 0.3 to 7 % by weight, with respect to the total weight of the composition, of oxidation dye precursor of ortho and/or para type and of coupler.

13. Process according to any one of Claims 2 to 12, characterised in that the composition (A) contains cationic, anionic, nonionic or amphoteric surface-active agents or their mixtures, in concentrations of between 0.5 and 55 % by weight with respect to the total weight of the composition; organic solvents in concentrations of between 1 and 40 % by weight with respect to the total weight of the composition; thickening agents in concentrations of between 0.1 and 5 % by weight with respect to the total weight of the composition; antioxidising agents in proportions of between 0.05 and 1.5 % by weight with respect to the total weight of the composition, direct dyes, fragrances, sequestering agents, film-forming agents, treatment agents, dispersing agents, conditioning agents, preserving agents, opacifying agents and any other agent generally used in dyeing compositions.

14. Process according to any one of Claims 2 to 13, characterised in that the compositions (A) and (B) are provided in the liquid, cream or gel form or any other suitable form, or can be packaged under pressure in aerosol containers in the presence of a propellant and to form foams.

15. Process according to Claim 1, characterised in that there is applied to keratinous fibres and more particularly human hair, in a first stage, a composition containing at least one para-aminophenol of formula (I), in a second stage, a composition containing 6-hydroxyindole and/or 7-hydroxyindole, and the colouring is developed using an oxidising agent present in this latter composition or else this oxidising agent is applied in a third stage, the pH of the mixture forming at the level of the fibre being greater than 7.

16. Oxidation dyeing process according to any one of Claims 1 to 15, characterised in that, after application to keratinous fibres, and more particularly human hair, the mixture is left exposed for 10 to 40 minutes, preferably 15 to 30 minutes, the hair is rinsed, is washed with the shampoo, is rinsed again and is dried.

17. Agent for dyeing keratinous fibres, in particular human hair, characterised in that it comprises at least two components, one of the components consisting of the composition (A) defined in any one of Claims 2 to 14 and the other consisting of the composition (B) also defined in any one of Claims 2 to 14, the pH of the compositions (A) and (B) being such that, after mixing in proportions of 90 to 10 % for the composition (A) and of 10 to 90 % for the composition (B), the resulting composition has a pH greater than 7.

18. Composition intended to be used for colouring keratinous fibres at basic pH, characterised in that it results from the mixing of 10 to 90 % of the composition (A) defined in any one of Claims 2 to 14 with 90 to 10 % of the composition (B) defined in any one of Claims 2 to 14 and in that it has a pH greater than 7.

19. Multi-compartment device or dyeing kit, characterised in that it comprises, in a first compartment, the composition (A) defined in any one of Claims 2 to 14 and, in a second compartment, the composition (B) defined in any one of Claims 2 to 14.

## Patentansprüche

1. Verfahren zur Färbung keratinischer Fasern, insbesondere menschlicher keratinischer Fasern wie der Haare,
dadurch **gekennzeichnet,**
daß man auf diese Fasern mindestens ein in Position 2 substituiertes p-Aminophenol der Formel: 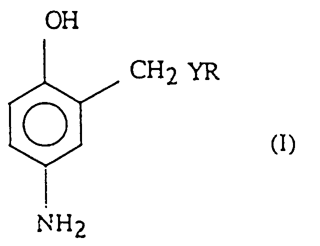 worin Y ein Sauerstoff- oder Schwefelatom und
R einen C₁₋₄-Alkyl-, Monohydroxy-C₁₋₄-alkyl- oder Polyhydroxy-C₂₋₄-alkyl-Rest darstellen, wobei deren Additionssalze mit einer Säure eingeschlossen sind, und 6-Hydroxyindol und/oder 7-Hydroxyindol aufbringt,
wobei die Farbe bei einem alkalischen pH-Wert und mit einem oxidierenden Mittel entwickelt wird.

2. Verfahren gemäß Anspruch 1,
dadurch **gekennzeichnet,**
daß man auf die keratinischen Fasern, insbesondere die menschlichen keratinischen Fasern, mindestens eine Zusammensetzung (A), die, in einem zur Färbung geeigneten Milieu, als Oxidationsfarbstoff-Vorstufenverbindung mindestens ein in Position 2 substituiertes p-Aminophenol der Formel (I) und 6-Hydroxyindol und/oder 7-Hydroxyindol als Kuppler enthält, aufbringt,
wobei die Farbe bei alkalischem pH-Wert und mit einem oxidierenden Mittel entwickelt wird, das in der Zusammensetzung (A) vorhanden ist oder, gleichzeitig oder nacheinander, in getrennter Form mittels einer Zusammensetzung (B) aufgebracht wird, die es in einem zur Färbung geeigneten Milieu enthält.

3. Verfahren gemäß Anspruch 1 oder 2,
dadurch **gekennzeichnet,**
daß das oxidierende Mittel aus Wasserstoffperoxid, Harnstoffperoxid, Alkalimetallbromaten und Persalzen ausgewählt ist.

4. Verfahren gemäß jedem der Ansprüche 1 bis 3,
dadurch **gekennzeichnet,**
daß in den Verbindungen der Formel (I) der Rest R einen Methyl-, Ethyl-, Propyl-, Isopropyl-, Butyl-, Isobutyl-, oder einen Mono- oder Polyhydroxyalkyl-Rest bedeutet, ausgewählt aus:
-CH₂-CH₂OH, -CH₂-CHOH-CH₂-OH, -CH₂-CHOH-CH₃.

5. Verfahren gemäß jedem der Ansprüche 1 bis 4,
dadurch **gekennzeichnet,**
daß die Verbindungen der Formel (I) aus den folgenden Verbindungen ausgewählt sind:
- 2-Methoxmethyl-4-aminophenol
- 2-Ethoxymethyl-4-aminophenol
- 2-Propoxymethyl-4-aminophenol
- 2-Isopropoxymethyl-4-aminophenol
- 2-(β-Hydroxyethoxymethyl)-4-aminophenol
- 2-Methylthiomethyl-4-aminophenol
- 2-(β-Hydroxyethylthiomethyl)-4-aminophenol
- 2-(β,γ-Dihydroxypropylthiomethyl)-4-aminophenol
und aus deren Salzen.

6. Verfahren gemäß jedem der Ansprüche 1 bis 5,
dadurch **gekennzeichnet,**
daß man auf die keratinischen Fasern 6-Hydroxyindol als Kuppler aufbringt.

7. Verfahren gemäß jedem der Ansprüche 1 bis 6,
dadurch **gekennzeichnet,**
daß der pH-Wert von der oder den auf die keratinischen Fasern aufgebrachten Zusammensetzung(en) so eingestellt ist, daß der Wert des auf den Fasern endgültig vorherrschenden pH-Wertes 8 bis 11 beträgt.

8. Verfahren gemäß jedem der Ansprüche 2 bis 7,
dadurch **gekennzeichnet,**
daß die Zusammensetzung (A) 0,05 bis 3,5 Gew.-%, bezogen auf das Gesamtgewicht der Zusammensetzung, mindestens einer Verbindung der Formel (I) enthält.

9. Verfahren gemäß jedem der Ansprüche 2 bis 8,
dadurch **gekennzeichnet,**
daß die Zusammensetzung (A) 0,01 bis 4 Gew.-% 6-Hydroxyindol und/oder 7-Hydroxyindol enthält, bezogen auf das Gesamtgewicht der Zusammensetzung.

10. Verfahren gemäß jedem der Ansprüche 2 bis 9,
dadurch **gekennzeichnet,**
daß die Zusammensetzung (A) außer dem p-Aminophenol der oben definierten Formel (I) weitere Oxidationsfarbstoff-Vorstufenverbindungen vom o- oder p-Typ enthält, die aus p-Phenylendiaminen, p-Aminophenolen, die sich von denen der Formel (I) unterscheiden, aus heterocyclischen Vorstufenverbindungen aus para-Derivaten des Pyridins oder Pyrimidins, o-Aminophenolen, o-Phenylendiaminen und aus Bisphenylalkylendiaminen ausgewählt sind.

11. Verfahren gemäß jedem der Ansprüche 2 bis 10,
dadurch **gekennzeichnet,**
daß die Zusammensetzung (A) außer dem 6-Hydroxyindol und/oder 7-Hydroxyindol weitere Kuppler wie m-Aminophenole, m-Phenylendiamin, m-Acylaminophenole, m-Ureidophenole, m-Carbalkoxyaminophenole, α-Naphthol und Kuppler mit einer aktiven Methylen-Gruppe enthält.

12. Verfahren gemäß jedem der Ansprüche 2 bis 11,
dadurch **gekennzeichnet,**
daß die Zusammensetzung (A) 0,3 bis 7 Gew.-% Oxidationsfarbstoff-Vorstufenverbindung des o- und/oder p-Typs und Kuppler enthält, bezogen auf das Gesamtgewicht der Zusammensetzung.

13. Verfahren gemäß jedem der Ansprüche 2 bis 12,
dadurch **gekennzeichnet,**
daß die Zusammensetzung (A) kationische, anionische, nicht-ionische oder amphotere oberflächenaktive Mittel oder deren Mischungen in Konzentrationen von 0,5 bis 55 Gew.-%, bezogen auf das Gesamtgewicht der Zusammensetzung, organische Lösungsmittel in Konzentrationen von 1 bis 40 Gew.-%, bezogen auf das Gesamtgewicht der Zusammensetzung, Verdickungsmittel in Konzentrationen von 0,1 bis 5 Gew.-%, bezogen auf das Gesamtgewicht der Zusammensetzung, Antioxidantien in Mengenanteilen von 0,05 bis 1,5 Gew.-%, bezogen auf das Gesamtgewicht der Zusammensetzung, Direktfarbstoffe, Parfüm-Produkte, Sequestriermittel, filmbildende Mittel, Behandlungsmittel, Dispergiermittel, Konditioniermittel, Konservierungsmittel, opak machende Mittel sowie jedes weitere gewöhnlich in Färbezusammensetzungen verwendete Mittel enthält.

14. Verfahren gemäß jedem der Ansprüche 2 bis 13,
dadurch **gekennzeichnet,**
daß die Zusammensetzungen (A) und (B) in Form einer Flüssigkeit, Creme, eines Gels oder in jeder weiteren Form vorliegen oder unter Druck in einem Aerosol-Fläschchen in Gegenwart eines Treibmittels zubereitet sein und Schäume bilden können.

15. Verfahren gemäß Anspruch 1,
dadurch **gekennzeichnet,**
daß man auf die keratinischen Fasern und insbesondere die menschlichen Haare, in einer ersten Stufe, eine Zusammensetzung, die mindestens ein p-Aminophenol der Formel (I) enthält, und in einer zweiten Stufe eine Zusammensetzung aufbringt, die das 6-Hydroxyindol und/oder das 7-Hydroxyindol enthält, und daß man die Färbung mit einem oxidierenden Mittel entwickelt, das in dieser letzteren Zusammensetzung vorliegt, oder daß man dieses oxidierende Mittel auch in einer dritten Stufe aufbringt, wobei der sich auf der Faser bildende pH-Wert der Mischung oberhalb 7 liegt.

16. Verfahren zur Oxidationsfärbung gemäß jedem der Ansprüche 1 bis 15,
dadurch **gekennzeichnet,**
daß man nach Aufbringung auf die keratinischen Fasern und insbesondere auf die menschlichen Haare die Mischung 10 bis 40 und vorzugsweise 15 bis 30 min lang verweilen läßt, die Haare spült, sie unter Schamponieren wäscht, erneut spült und trocknet.

17. Mittel zur Färbung keratinischer Fasern, insbesondere der menschlichen Haare,
dadurch **gekennzeichnet,**
daß es mindestens zwei Bestandteile aufweist, wobei der eine der Bestandteile aus der in jedem der Ansprüche 2 bis 14 definierten Zusammensetzung (A) und der andere aus der ebenfalls in jedem der Ansprüche 2 bis 14 definierten Zusammensetzung (B) zusammengesetzt sind, und wobei der pH-Wert der Zusammensetzungen (A) und (B) so eingestellt ist, daß nach Vermischung in Mengenanteilen von 90 bis 10 % für die Zusammensetzung (A) und von 10 bis 90 % für die Zusammensetzung (B) die entstandene Zusammensetzung einen pH-Wert von mehr als 7 aufweist.

18. Zusammensetzung zur Verwendung zur Färbung keratinischer Fasern bei basischem pH-Wert,
dadurch **gekennzeichnet,**
daß sie aus der Vermischung von 10 bis 90 % der in jedem der Ansprüche 2 bis 14 definierten Zusammensetzung (A) mit 90 bis 10 % der in jedem der Ansprüche 2 bis 14 definierten Zusammensetzung (B) entsteht und einen pH-Wert von mehr als 7 aufweist.

19. Vorrichtung aus mehreren Bestandteilen oder Kit zur Färbung,
dadurch **gekennzeichnet,**
daß sie in einem ersten Teil die in jedem der Ansprüche 2 bis 14 definierte Zusammensetzung (A) und in einem zweiten Teil die in jedem der Ansprüche 2 bis 14 definierte Zusammensetzung (B) umfassen.
